# EUROPÄISCHE PATENTSCHRIFT

(11) **EP 1 854 506 B1**
(45) Veröffentlichungstag und Bekanntmachung des Hinweises auf die Patenterteilung: **11.03.2009**
(21) Anmeldenummer: 07106645.0
(22) Anmeldetag: 20.04.2007
(51) Int. Cl.: A61N 5/10, A61B 6/08, G01B 11/27

(54) **Überprüfung der Positioniergenauigkeit eines Patiententisches in Bezug auf ein Isozentrum**
Verification of position accuracy of a patient table relative to isocentre
Vérification de la précision du positionnement d'une table pour patient en référence à un isocentre

(30) Priorität: 09.05.2006 DE 102006021632
(43) Veröffentlichungstag der Anmeldung: 14.11.2007
(73) Patentinhaber: SIEMENS AKTIENGESELLSCHAFT, 80333 München (DE)
(72) Erfinder: Gunzert-Marx, Konstanze, 91054, Erlangen (DE); Use, Tim, 90469, Nürnberg (DE)

(56) Entgegenhaltungen:
- WO-A-01/76692
- WO-A-20/06055770
- DE-A1- 3 543 786
- US-A- 5 142 559
- US-A- 5 612 533
- US-A1- 2004 228 435

## Beschreibung

Die Erfindung betrifft ein medizintechnisches Gerät, insbesondere ein Strahlentherapiegerät, mit einem in einem Isozentrum positionierbaren Patiententisch und mit einem optischen Koordinatenanzeigesystem, welches mindestens eine zur Emission eines Prüfstrahles vorgesehene Strahlenquelle aufweist, insbesondere einen Laserstrahler. Die Erfindung betrifft weiterhin einer Prüfkörper sowie ein Verfahren zur Überprüfung der Positioniergenauigkeit eines Patiententisches in Bezug auf ein Isozentrum eines medizintechnischen Geräts.

Die korrekte Positionierung des Patienten relativ zu einem medizintechnischen Gerät ist für die Therapie eines Patienten von großer Bedeutung. Besonders in der Partikeltherapie, wo die Positioniergenauigkeiten des Partikelstrahls um ein mehrfaches besser sind als bei der konventionellen Photonenbestrahlung oder der Protonenbestrahlung mit Scattering, ist die exakte Positionierung des zu bestrahlende Gewebes im Isozentrum des Strahlentherapiegeräts von elementarer Bedeutung. Hierbei werden an die Positioniergenauigkeit des Patiententisches und deren Reproduzierbarkeit sehr hohe Anforderungen gestellt, um jegliche Fehler so gering wie möglich zu halten.

Grundvoraussetzung für die korrekte Positionierung des Patienten ist die exakte Bestimmung eines Raumkoordinatensystems. Typischerweise wird ein Laserkoordinatenanzeigesystem bei der Installation und einmal jährlich mit einem Theodoliten eingemessen. Das Laserkoordinatenanzeigesystem umfasst üblicherweise mindestens drei orthogonal zueinander ausgerichtete, linienförmige Strahlfächer, deren Schnittpunkt den Ort des Isozentrums angibt. Zur Überprüfung der Positioniergenauigkeit werden auf dem Patiententisch Prüfkörper, die s.g. Phantome mit Markierungen an festgelegten Positionen montiert.

Mit Hilfe dieser Prüfkörper können Ungenauigkeiten in der Position gegenüber dem Laserkoordinatenanzeigesystem festgestellt und korrigiert werden. Die Positionierung der Prüfkörper gegenüber dem Laserkoordinatenanzeigesystem erfolgt in der Regel visuell durch das zuständige Bedienpersonal.

Aus der US 5,142,559 ist ein medizinisches Gerät nach dem Oberbegriff des Anspruchs 1 bekannt. Aus dieser Druckschrift ist eine Detektoranordnung zu entnehmen, die zu einer Positionsverifikation eines Lasers eines Bestrahlungssystems eingesetzt wird. Die Detektoranordnung weist eine flache Oberseite auf, auf der vier Gruppen von Photodioden angeordnet sind.

Der Erfindung liegt die Aufgabe zugrunde, die Überprüfung der Positioniergenauigkeit eines Patiententisches eines medizintechnischen Gerätes in einem Laserkoordinatenanzeigesystem zu vereinfachen.

Diese Aufgabe wird erfindungsgemäß gelöst durch ein medizintechnisches Gerät, insbesondere ein Strahlentherapiegerät, mit einem in einem Isozentrum positionierbaren Patiententisch und mit einem optischen Koordinatenanzeigesystem, welches mindestens eine zur Emission eines Prüfstrahles vorgesehene Strahlenquelle aufweist, insbesondere einen Laserstrahler, wobei zur Überprüfung der Positioniergenauigkeit des Patiententisches ein Prüfkörper zur Strahlendetektierung mit zumindest einer aus einer Reihe Photozellen aufgebauten Photozeile vorgesehen ist, deren Position mit der des Patiententisches korreliert ist, wobei der Prüfkörper nach Art eines auf dem Kopf stehenden Tisches mit einer plattenförmigen Basis und zumindest einem rechtwinklig hierzu angeordneten Pfeiler ausgebildet ist, wobei auf der Basis zumindest zwei unter einem Winkel zueinander angeordnete Photozeilen und eine weitere Photozeile am Pfeiler vorgesehen sind.

Hierbei wird der Laserstrahl auf die Photozeile gerichtet und derart angesteuert und bewegt, dass er einen Strahlenfächer bildet, der nach Art einer Laserlinie oder Prüfstrahl die Photozeile schneidet. Die Photozellen, die mit dem Prüfstrahl belichtet werden, liefern einer Steuereinrichtung des medizintechnischen Geräts ein Signal, das rechnerisch ausgewertet wird, um Daten über die genauen Positionskoordinaten und eventuell die Orientierung des Prüfkörpers zu erhalten.

Die Detektierung des von der Strahlenquelle emittierten Strahls, insbesondere Laserstrahls, mittels einer aus aneinander gereihten Photozellen aufgebauten Photozeile hat den Vorteil, dass eine unmittelbare visuelle Kontrolle durch das Bedienpersonal des medizintechnischen Gerätes entbehrlich wird. Damit sind entsprechende Fehlerquellen eliminiert, wobei auch die Genauigkeit der Überprüfung des Koordinatensystems bei Verwendung entsprechend hochauflösender Photozellen erhöht ist. Zudem sind die von der Photozeile aufgenommenen Signale automatisch durch die zur Ansteuerung des medizintechnischen Gerätes vorgesehene Steuereinrichtung weiterverarbeitbar, wodurch die Genauigkeit verbessert und die Sicherheit erhöht wird. Somit wird eine Automatisierung und Objektivierung des bisherigen Überprüfungsverfahrens erreicht.

Hierbei ist der Prüfkörper in einer ersten bevorzugten Variante ein gesondertes geometrisches Objekt, das getrennt vom Patiententisch über eine Verstelleinrichtung des Patiententisches, beispielsweise ein Roboterarm, im Isozentrum positioniert wird. Seine korrekten Koordinaten bezüglich des Isozentrums werden gespeichert, um den Patiententisch jederzeit später mit der Verstelleinrichtung in eine definierte Position relativ zum Isozentrum verfahren zu können. Alternativ ist der gesonderte Prüfkörper auf dem Patiententisch montiert, wodurch eine direkte Korrelation zwischen seinen Koordinaten und denen des Patiententisches aufgestellt wird. In einer weiteren Ausführungsvariante ist der Prüfkörper Teil des Patiententisches und umfasst mehrere Elemente, die an unterschiedlichen Positionen des Patiententisches befestigt oder im Patiententisch integriert sind.

Bevorzugt ist die Strahlausdehnung des Prüfstrahls derart relativ zur Photozeile bemessen, dass der Prüfstrahl lediglich auf einen kleinen Teil der Photozellen der Photozeile trifft. Hierbei wird in nur einem Teil der Photozellen ein Signal, insbesondere ein eine einstellbare Schwelle überschreitendes Signal erzeugt. Somit ist es möglich, eine Verschiebung des Signals entlang der Photozeile zu erfassen, die Informationen über Abweichungen der Position des Prüferkörpers vom Isozentrum liefert. Insbesondere werden bei einer ersten Justage des Prüfkörpers die Daten über die Lage des Signals entlang jeder einzelnen Photozeile als eine Nulllage gespeichert, und bei einer erneuten Überprüfung der Positioniergenauigkeit des Prüfkörpers werden die neu gewonnenen Daten mit der hinterlegten Nulllage des Signals in jeder Photozeile verglichen.

Gemäß einer bevorzugten Ausgestaltung sind mehrere einen Winkel, insbesondere einen rechten Winkel, miteinander einschließende Photozeilen vorgesehen. Somit kann die Position des Prüfkörpers zwei- oder dreidimensional erfasst werden. Insbesondere ist der Prüfkörper derart positioniert, dass die Positionierzeilen im Wesentlichen entlang der Achsen des Koordinatenanzeigesystems verlaufen. Dabei ist die Auswertung der exakten Position des Prüfkörpers im Koordinatenanzeigesystem besonders einfach.

Gemäß einer weiteren bevorzugten Ausgestaltung sind mehrere parallel zueinander angeordnete und in Strahlrichtung des Prüfstrahles voneinander beabstandete Photozeilen vorgesehen. Dank dieser Ausgestaltung kann nicht nur eine Verschiebung des Prüfkörpers vom Isozentrum, sondern auch eine Verdrehung des Prüfkörpers bezüglich des Koordinatenanzeigesystems detektiert werden, wenn beispielsweise zwei parallele, gegenüberliegende Photozeilen mit dem Prüfstrahl belichtet werden.

Die Erfassung einer Verdrehung des Prüfkörpers durch die parallele Anordnung der Photozeilen ist durch die Größe der Photozeilen beschränkt, so dass meist nur Abweichungen um wenige Grad detektierbar sind. Um weitere Winkelstellungen zu überprüfen, die auf eine größere Verdrehung gegenüber der Achsen des Koordinateanzeigensystems hindeuten, sind mehrere Photozeilen in einer Ebene erforderlich. Somit sind gemäß einer bevorzugten Weiterbildung mehrere Photozeilen vorgesehen, die auf einer gedachten Kreislinie angeordnet sind. Durch eine kreis- oder bogenförmige Anordnung mehrer Photozeilen kann eine Abweichung um einen besonders großen Winkelbetrag erfasst werden.

Vorteilhafterweise weist der Prüfkörper ein Verbindungselement zur präzisen Verbindung mit dem Patiententisch auf. Durch die präzise, wiederholbare Verbindung des Prüfkörpers mit dem Patiententisch ist auch die Position des Tisches im Koordinatenanzeigesystem definiert.

Zweckdienlicherweise ist der Prüfkörper an einer Kupplungsstelle der insbesondere als Roboter ausgeführte Verstelleinrichtung des Patiententisches koppelbar. Insbesondere ist der Prüfkörper über eine Werkzeugwechseleinheit direkt mit der Verstelleinrichtung koppelbar. Nach der Positionierung des Prüfkörpers im Isozentrum und der Speicherung der Koordinaten des Isozentrums wird der Prüfkörper mit Hilfe der Werkzeugwechseleinheit gegen den Patiententisch ausgetauscht und der Tisch kann in eine bezüglich des Isozentrums definierte Position verfahren werden.

Die Aufgabe wird weiterhin erfindungsgemäß gelöst durch einen Prüfkörper zur Überprüfung der Positioniergenauigkeit eines Patiententisches in Bezug auf ein Isozentrum eines medizintechnischen Geräts in einem Koordinatenanzeigesystem, wobei der Prüfkörper nach Art eines auf dem Kopf stehenden Tisches mit einer plattenförmigen Basis und zumindest einem rechtwinklig hierzu angeordneten Pfeiler ausgebildet ist, wobei auf der Basis zumindest zwei unter einem Winkel zueinander angeordnete Photozeilen und eine weitere Photozeile am Pfeiler vorgesehen sind und wobei die Photozeilen aus einer Reihe Photozellen aufgebaut sind.

Die Aufgabe wird ferner erfindungsgemäß gelöst durch ein Verfahren zur Überprüfung der Positioniergenauigkeit eines Patiententisches in Bezug auf ein Isozentrum eines medizintechnischen Geräts in einem Koordinatenanzeigesystem, bei dem ein Prüfkörper in eine Prüfposition gebracht wird, wobei der Prüfkörper nach Art eines auf dem Kopf stehenden Tisches mit einer plattenförmigen Basis und zumindest einem rechtwinklig hierzu angeordneten Pfeiler ausgebildet ist, wobei auf der Basis zumindest zwei unter einem Winkel zueinander angeordnete aus einer Reihe Photozellen aufgebauten Photozeilen und eine weitere aus einer Reihe Photozellen aufgebauten Photozeile am Pfeiler vorgesehen sind, die Photozeile mit einem von einer Strahlenquelle des Koordinatenanzeigesystems emittierten Prüfstrahl bestrahlt wird und in Abhängigkeit von dem von der Photozeile aufgenommenen Signal die Position des Prüfkörpers relativ zum Isozentrum ermittelt wird.

Die im Hinblick auf das medizintechnische Gerät aufgeführten Vorteile und bevorzugen Ausführungsformen lassen sich sinngemäß auf den Prüfkörper und das Verfahren übertragen.

Vorzugsweise wird die Lage des Signals der Photozeile mit einer Nulllage verglichen, die bei einer Justage des Prüfkörpers im Koordinatenanzeigesystem definiert ist. Somit werden Verschiebungen und eventuell Verdrehungen des Prüfkörpers detektiert.

Um eine dreidimensionale Überprüfung der Positioniergenauigkeit des Prüfkörpers bzw. des Patiententisches zu ermöglichen, wird der Prüfkörper bevorzugt aus mehreren Richtungen, insbesondere aus drei Richtungen parallel zu den Achsen des Koordinatenanzeigesystems bestrahlt.

Nach einer bevorzugten Variante wird der Prüfkörper zur Ermittlung der Position relativ zum Isozentrum bewegt und das von der Photozeile aufgenommene Signal wird ausgewertet. Durch die translatorischen oder rotatorischen Bewegungen werden die Abweichungen von der korrekten Position ausgeglichen, bis die Nulllage des Signals auf den Photozeilen erreicht wird.

Die Bewegungen des Prüfkörpers können außerdem dazu dienen, Informationen über die Orientierung des Prüfkörpers zu gewinnen. Insbesondere in der Ausführungsform, bei der lediglich eine Photozeile pro Koordinatenrichtung vorgesehen ist, kann eine Abweichung des Signals von der Nulllage sowohl auf eine translatorische Verschiebung als auch auf eine Verdrehung des Prüfkörpers hindeuten. Um festzustellen, welcher der beiden Fälle vorliegt, wird der Prüfkörper in einer Ebene um das angenommene Isozentrum beispielweise um 90° gedreht und seine Position wird erneut überprüft. Wenn nun eine Verschiebung des Signals der einzelnen Photozeilen gegenüber seiner vorherigen Lage erfassbar ist, ist der Drehpunkt des Prüfkörpers in dieser Ebene ungleich mit dem Isozentrum.

Ausführungsbeispiele der Erfindung werden nachfolgend anhand der Zeichnung erläutert. Hierin zeigen jeweils in schematischer Darstellung:
- FIG 1: ein medizintechnisches Gerät mit einem optischen Koordinatenanzeigesystem und einem Prüfkörper,
- FIG 2: eine Photozeile des Prüfkörpers gemäß FIG 1 sowie beispielhafte Verläufe damit aufgenommener Signale,
- FIG 3: eine erste Orientierung mehrerer Photozeilen des Prüfkörpers gemäß FIG 1 bezüglich zweier Prüfstrahlen des Koordinatenanzeigesystems,
- FIG 4: eine zweite Orientierung mehrerer Photozeilen des Prüfkörpers gemäß FIG 1 bezüglich zweier Prüfstrahlen des Koordinatenanzeigesystems,
- FIG 5: eine Draufsicht auf einen Prüfkörper und
- FIG 6: eine perspektivische Darstellung des Prüfkörpers gemäß FIG 5, und
- FIG 7a-7c: Draufsichten auf einen weiteren Prüfkörper in drei unterschiedlichen Ausrichtungen zum Koordinatenanzeigesystem.

Einander entsprechende Teile und Parameter sind in allen Figuren mit den gleichen Bezugszeichen gekennzeichnet.

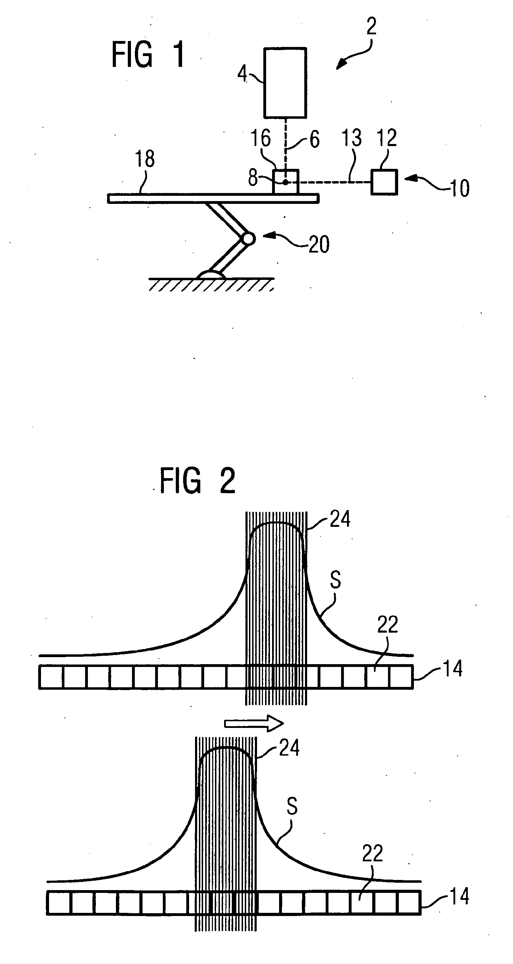

Ein in FIG 1 symbolisiert dargestelltes medizintechnisches Gerät 2 umfasst einen Partikelstrahler 4, beispielsweise ein Protonenstrahler oder Schwerionenstrahler, der im Betrieb einen Partikelstrahl 6 emittiert, welcher bei der Durchführung einer Strahlentherapie in einem Isozentrum 8 auf zu bestrahlendes Gewebe eines nicht dargestellten Patienten trifft.

Das medizintechnische Gerät 2 weist ein optisches Koordinatenanzeigesystem 10 auf, das einen Laserstrahler als Strahlenquelle 12 umfasst. Zur Strahlendetektierung eines von dem Laserstrahler 12 emittierten Laserstrahls 13 sind hier nicht dargestellten Photozeilen 14 vorgesehen (vgl. FIG 2 bis FIG 6), die auf einem Prüfkörper 16 angebracht sind. In FIG 1 ist schematisch lediglich eine einzige Strahlenquelle 12 dargestellt, welche die Position des Prüfkörpers 16 in eine Richtung des Koordinatenanzeigesystems 10 bestimmt. Tatsächlich sind mindestens drei die Achsen eines Koordinatensystems beschreibende Laserstrahler 12 vorhanden.

Der Prüfkörper 16 dient zur Überprüfung der Positioniergenauigkeit eines Patiententisch 18 des medizintechnischen Geräts 2 im Koordinatenanzeigesystem 10. In diesem Ausführungsbeispiel ist der Prüfkörper 16 ein gesondertes Objekt, das lösbar mit dem Patiententisch 18 verbunden ist und mittelbar über eine Verstelleinrichtung 20 des Tisches 18 verfahrbar ist. Die Positionierung des Prüfkörpers 16 auf dem Patiententisch 18 ist hoch präzise und reproduzierbar, so dass eine eindeutige Korrelation zwischen der Position des Prüfkörpers 16 und der das Patiententisches 18 im Koordinatenanzeigesystem 10 gewährleistet ist.

Sämtliche Laserstrahler 12 und der Prüfkörper 16 sind datentechnisch mit einer hier nicht dargestellten Steuereinrichtung des medizintechnischen Gerätes 2 verbunden. Die durch die Strahlendetektierung erhaltenen Daten werden in der Steuereinrichtung ausgewertet, so dass ermittelt wird, ob es Abweichungen der Position des Prüfkörpers 16 bezüglich des Isozentrums 8 gibt. Wenn Abweichungen vorliegen werden sie in diesem Ausführungsbeispiel korrigiert, indem die Steuereinrichtung über die Verstelleinrichtung 20 die Position des Tisches 18 und damit des Prüfkörpers 16 entsprechend ändert.

Der Prüfkörper 16 kann auch aus mehreren Elementen bestehen, die entfernt voneinander auf dem Patiententisch 18 montiert sind oder ein integraler Bestandteil des Tisches 18 sind. Alternativ kann der Prüfkörper 16 über eine Werkzeugwechseleinheit, ein sogenanntes Tool Changer, direkt mit der Verstelleinrichtung 20 verbunden sein. Nach der Überprüfung der Positioniergenauigkeit relativ zum Isozentrum 8 wird in diesem Fall mit Hilfe des Tool Changers der Prüfkörper 16 durch den Tisch 18 ersetzt und der Tisch 18 kann wiederholt in gewünschten definierten Positionen gegenüber dem Isozentrum 8 verfahren werden.

In FIG 2 ist eine Photozeile 14 des Prüfkörpers 16 gezeigt. Die Photozeile 14 ist aus mehreren geometrisch in Reihe angeordneten Photozellen 22 zusammengesetzt. Als Photozellen 22 werden beispielsweise CCD-Zellen (charge coupled device) verwendet. Ebenso sind jegliche sonstige photosensitive Sensoren geeignet.

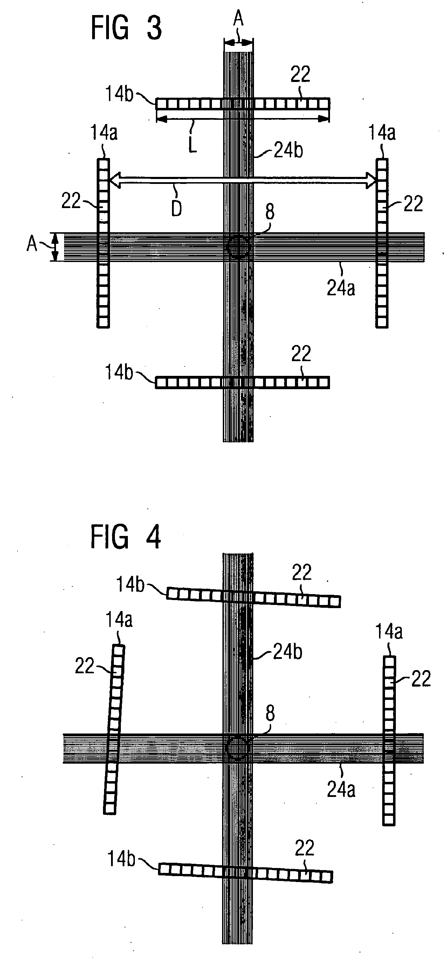

In FIG 2 ist zusätzlich zu einer Photozeile 14 ein von dieser geliefertes elektrisches Signal S bei zwei unterschiedlichen Belichtungen mit einem Laserstrahl 13 der Strahlungsquelle 12 dargestellt. Der Laserstrahl 13 bildet einen Strahlfächer, der nach Art einer Laserlinie als Prüfstrahl 24 die Photozeile 14 schneidet. Die Strahlausdehnung A (FIG 3) ist derart bemessen, dass der Prüfstrahl 24 lediglich auf einen kleinen Teil der Photozellen 22 der Photozeile 14 trifft.

Die Verschiebung des Prüfstrahls 24 von einer ersten Einstrahlposition - in FIG 2 unten dargestellt - in eine zweite Einstrahlposition ist durch einen Pfeil veranschaulicht. Während in den Photozellen 22, die direkt durch den Prüfstrahl 24 belichtet werden, eine hohe Signalstärke erzeugt wird, fällt das Signal S mit zunehmender Entfernung von dem Zentrum des Prüfstrahls 24 ab. Durch die Auswertung, welche der Photozellen 22 vom Prüfstrahl 24 bestrahlt wird, wird die Position des Prüfstrahls 24 relativ zum Prüfkörper 16 ermittelt. Bevorzugt werden nur solche Photozellen als vom Prüfstrahl 24 bestrahlt ausgewertet, deren abgegebener Signalwert einen insbesondere einstellbaren Schwellwert übersteigt.

In einer Photozelle 22 als lichtempfindlicher Photodiode entsteht bei Belichtung eine Ladung, die zur Intensität des auftreffenden Lichts proportional ist. In einer ersten Weise der Signalverarbeitung wird lediglich erfasst, ob die Intensität des von der Photozelle 22 detektierten Lichts die vorzugsweise einstellbare Schwelle überschreitet. Der Ort der Einstrahlung des Prüfstrahls 24 ist dann durch die Koordinaten der Photozelle 22 beziehungsweise - bei mehreren belichteten Photozellen 14 - durch die gemittelten Koordinaten der betreffenden Photozellen 14 definiert.

Nach einer alternativen Methode der Signalverarbeitung wird nicht nur die Überschreitung einer Schwelle, sondern - in feinerer Auflösung, insbesondere mit einer digitalen Skala - auch die Stärke des Signals S bei jeder belichteten Photozelle 22 erfasst. Auf diese Weise ist das Zentrum der Einstrahlung des Prüfstrahls 24 mit einer Genauigkeit bestimmbar, die die Ortsauflösung der einzelnen Photozellen 22, das heißt die Dimensionierung der typischerweise quadratischen Photozellen 22 in Erstreckungsrichtung der Photozeile 14 übertrifft.

Wie genau Informationen über die Position und Orientierung des Prüfkörpers 16 in einer zweidimensionalen Ebene erhalten werden, wird aus FIG 3 und FIG 4 ersichtlich. In FIG 3 sind zwei Photozeilen 14a parallel zueinander angeordnet, wobei der Abstand zwischen den gleichartigen Photozeilen 14a mit D angegeben ist. Zusätzlich sind zwei weitere Photozeilen 14b ebenfalls parallel zueinander angeordnet. Diese Photozeilen 14b sind orthogonal zu den Photozeilen 14a angeordnet, so dass die insgesamt vier Photozeilen 14a, 14b an den Seiten eines gedachten Rechtecks, insbesondere eines Quadrats angeordnet sind. In der Mitte dieses Quadrats befindet sich das Isozentrum 8, in welchem das mit dem Partikelstrahl 6 zu behandelnde Gewebe des Patienten zu positionieren ist.

Jedes Paar an Photozeilen 14a, 14b wird von einem Prüfstrahl 24a, 24b belichtet, welcher einen lang gestreckten rechteckigen Querschnitt aufweist und nach Art einer Laserlinie auf die Ebene der Photozeilen 14a, 14b auftrifft. Die in FIG 3 sichtbaren Prüfstrahlen 24a, 24b schneiden die zugeordneten Photozeilen 14a, 14b jeweils exakt rechtwinklig. Das Isozentrum 8 liegt im Schnittpunkt der beiden Prüfstrahlen 24a, 24b. Jeder Prüfstrahl 24a, 24b schneidet die zugeordnete Photozeile 14a, 14b nur auf einem kleineren Teil ihrer Länge L im dargestellten Ausführungsbeispiel weniger als einem Viertel der Länge L.

Die FIG 4 zeigt ebenfalls die Photozeilen 14a, 14b in der Anordnung nach FIG 3. Lediglich die Orientierung des Prüfkörpers 16 und somit der Photozeilen 14a, 14b unterscheidet sich vom anhand der FIG 3 erläuterten Fall: Gemäß FIG 4 sind beide Paare an Photozeilen 14a, 14b nicht orthogonal zum jeweiligen Prüfstrahl 24a, 24b gerichtet.

Dies veranschaulicht, dass das Koordinatenanzeigesystem 10 nicht nur zur Detektierung von Verschiebungen, sondern auch zur quantitativen Erfassung von Verdrehungen des Prüfkörpers 16 bzw. der Photozeilen 14a, 14b relativ zu den entsprechenden Prüfstrahlen 24a, 24b geeignet ist. Je größer der Abstand D zwischen zueinander parallelen Photozeilen 14a ist, desto größer ist die Winkelauflösung des optischen Messsystems 10.

Vor Beginn der Bestrahlung des Patienten ist eine Justage des Laserkoordinatensystems 10 vorzunehmen, um die in FIG 3 dargestellte räumliche Korrelation zwischen den Prüfstrahlen 24a, 24b und den Photozeilen 14a, 14b zu erreichen. Dabei wird die Lage des Signals S über die einzelnen Photozellen 14a, 14b mit einer Nulllage verglichen, die bei einer Erstjustage gespeichert wurde. Abweichungen von der richtigen Position und Ausrichtung der Photozellen 14a, 14b, welche beispielsweise aus FIG 4 ersichtlich sind, werden automatisch beim Vergleichen der Lage des gewonnenen Signals S mit der Nulllage erkannt und angezeigt. Diese Abweichungen werden auch von der Steuereinrichtung des medizintechnischen Geräts 2 korrigiert, indem der Prüfkörper 16 in Abhängigkeit vom abgelesenen Signal S jeder Photozeile 14a, 14b entsprechend translatorisch oder rotatorisch über die Verstelleinrichtung 20 bewegt wird.

Anstelle der einzelnen Photozeilen 14a, 14b kann zur Strahlendetektierung ein Array an Photozellen 22 vorgesehen sein. Es ist auch möglich, die Photozeilen 14 kreis- oder bogenförmig anzuordnen, wodurch eine erhöhte Winkelsensitivität erreichbar ist.

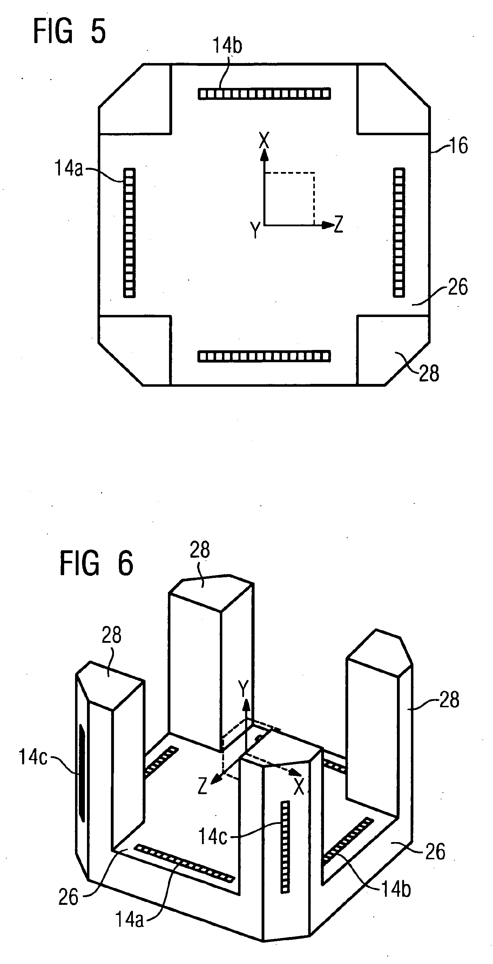

Die Anordnung der Photozeilen 14a, 14b auf dem Prüfkörper 16 in Bezug auf die X-, Y-, und Z-Achse des Koordinatenanzeigesystems 10 ist in FIG 5 und FIG 6 dargestellt. Der Prüfkörper 16 ist in dieser bevorzugten Ausgestaltung nach Art eines auf dem Kopf stehenden Tisches ausgebildet. Er weist eine Basis 26 auf, die im Wesentlichen in der horizontalen X-Z-Ebene des Koordinatenanzeigesystems 10 liegt. In dem vorliegenden Ausführungsbeispiel weist der Prüfkörper 16 auf einer Unterseite der Basis 26 ein hier nicht näher dargestelltes Verbindungselement, über welches eine hoch präzise Verbindung mit dem Tisch 18 herstellbar ist.

Zur Erfassung von Abweichungen der Position des Prüfkörpers 16 entlang der vertikalen Y-Achse sind in diesem Ausführungsbeispiel senkrecht zur Basis 26 vier Pfeiler 28 vorgesehen. Auf jedem der Pfeiler 28 ist eine Photozeile 14c parallel zur Y-Achse angeordnet. Die Photozeilen 14c werden von einem hier nicht dargestellten Prüfstrahl geschnitten, der sich in einer Ebene ausbreitet, die im Wesentlichen parallel zur Basis 26 ist. Zur Überprüfung der Positioniergenauigkeit des Prüfkörpers 16 würden auch zwei Pfeiler 28 ausreichen, auf denen zwei Photozeilen 14c derart angeordnet sind, dass beide Photozeilen 14c von einer Seite mit dem Prüfstrahl geschnitten werden können. In diesem Ausführungsbeispiel sind jedoch vier Pfeiler 28 vorgesehen, damit die Positioniergenauigkeit von allen vier Seiten in der X-Z-Ebene überprüft werden kann.

Durch die Anordnung von mindestens zwei Photozeilen 14c parallel zur Y-Achse, die vorzugsweise symmetrisch verlaufen und den gleichen Abstand (+X, -X) vom Isozentrum 8 aufweisen, können neben einer Rotation des Prüfkörpers 16 bzw. Patiententisches 18 im Isozentrum 8 auch Roll und Tilt, d.h. die Rotationen um die Z-Achse und um die X-Achse überprüft werden.

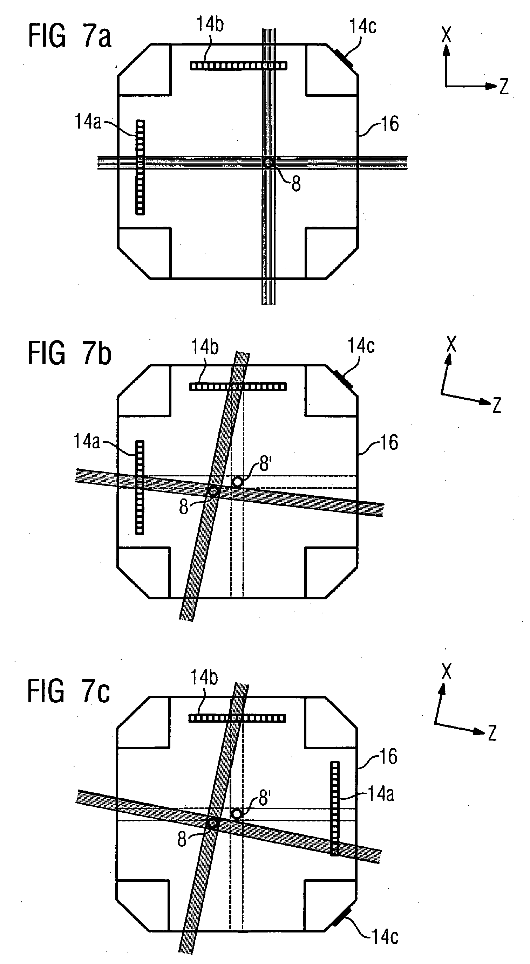

Alternativ zu den parallelen Photozeilenpaaren kann nur jeweils eine Photozeile 14a, 14b, 14c parallel zu den Achsen des Koordinatenanzeigesystems 10 vorgesehen sein. Mittels dieser Ausführungsvariante können insbesondere Verschiebungen des Prüfkörpers 16 erfasst werden, wie sie beispielsweise in FIG 7a gezeigt ist.

Bei nur einer Photozeile 14a, 14b, 14c in jeder Richtung kann eine Verdrehung des Prüfkörpers 16 nicht automatisch erkannt werden, denn eine geänderte Lage des Signals S auf den Photozeilen 14a, 14b kann sowohl auf eine Verschiebung als auch auf eine Verdrehung des Prüfkörpers 16 hindeuten. Außerdem kann der Prüfkörper 16 verdreht sein, auch wenn die Lage des Signals S auf beiden Photozeilen 14a, 14b mit der Nulllage übereinstimmt, wie es in FIG 7b gezeigt ist. Um festzustellen, ob eine Verdrehung vorliegt, wird der Prüfköper 16 um 90° z.B. im Uhrzeigersinn in der X-Z-Ebene um den Drehpunkt 8' rotiert. Der Punkt 8' ist der Schnittpunkt zweier Geraden, die senkrecht zu den Photozeilen 14a, 14b sind und die Photozeilen 14a, 14b in der Nulllage schneiden, somit wird im Drehpunkt 8' das Isozentrum 8 vermutet.

Die Ausrichtung des Prüfkörpers 16 nach der Rotation um 90° im Uhrzeigersinn ist in FIG 7c dargestellt. Eine erneute Überprüfung der Lage des Signals S auf den Photozeilen 14a, 14 b ergibt eine Verschiebung des Signals S entlang der X-Achse. Dies bedeutet, dass der Drehpunkt 8' des Prüfkörpers 16 in der X-Z-Ebene ungleich mit dem Isozentrum 8 ist. Eine weitere Rotation um 90° im Uhrzeigersinn würde ebenfalls eine Verschiebung des Signals S Entlang der Z-Achse ergeben. Mit den hierbei gewonnen Daten kann die tatsächliche Lage des Isozentrums 8 bestimmt werden und die Verdrehung des Prüfkörpers 16 in der X-Z-Ebene wird direkt von der Steuereinrichtung korrigiert.

## Patentansprüche

1. Medizintechnisches Gerät (2), insbesondere Strahlentherapiegerät, mit einem in einem Isozentrum (8) positionierbaren Patiententisch (18) und mit einem optischen Koordinatenanzeigesystem (10), welches mindestens eine zur Emission eines Prüfstrahles vorgesehene Strahlenquelle (12) aufweist, insbesondere einen Laserstrahler, wobei zur Überprüfung der Positioniergenauigkeit des Patiententisches (18) ein Prüfkörper (16) zur Strahlendetektierung mit aus einer Reihe Photozellen (22) aufgebauten Photozeilen (14) vorgesehen ist, deren Position mit der des Patiententisches (18) korreliert ist,
**dadurch gekennzeichnet, dass** der Prüfkörper (16) nach Art eines auf dem Kopf stehenden Tisches mit einer plattenförmigen Basis (26) und zumindest einem rechtwinklig hierzu angeordneten Pfeiler (28) ausgebildet ist, wobei auf der Basis (26) zumindest zwei unter einem Winkel zueinander angeordnete Photozeilen (14a, 14b) und eine weitere Photozeile (14c) am Pfeiler (28) vorgesehen sind.

2. Medizintechnisches Gerät (2) nach Anspruch 1,
**dadurch gekennzeichnet, dass** die Strahlausdehnung (A) des Prüfstrahls (24) derart relativ zur Photozeile (14) bemessen ist, dass der Prüfstrahl (24) lediglich auf einen Teil der Photozellen (22) der Photozeile (14) trifft.

3. Medizintechnisches Gerät (2) nach Anspruch 1 oder 2,
**dadurch gekennzeichnet, dass** die Photozeilen (14a, 14b, 14c) einen rechten Winkel miteinander einschließen.

4. Medizintechnisches Gerät (2) nach einem der Ansprüche 1 bis 3, **dadurch gekennzeichnet, dass** mehrere parallel zueinander angeordnete Photozeilen (14a, 14b, 14c) vorgesehen sind.

5. Medizintechnisches Gerät (2) nach Anspruch 3,
**dadurch gekennzeichnet, dass** die Photozeilen (14) auf einer gedachten Kreislinie angeordnet sind.

6. Medizintechnisches Gerät (2) nach einem der vorhergehenden Ansprüche,
**dadurch gekennzeichnet, dass** der Prüfkörper (16) ein Verbindungselement zur präzisen Verbindung mit dem Patiententisch (18) aufweist.

7. Medizintechnisches Gerät (2) nach einem der Ansprüche 1 bis 5,
**dadurch gekennzeichnet, dass** der Prüfkörper (16) an einer Kupplungsstelle einer Verstelleinrichtung (20) des Patiententisches (18) koppelbar ist.

8. Prüfkörper (16) zur Überprüfung der Positioniergenauigkeit eines Patiententisches (18) in Bezug auf ein Isozentrum (8) eines medizintechnischen Geräts (2) in einem Koordinatenanzeigesystem (10), der nach Art eines auf dem Kopf stehenden Tisches mit einer plattenförmigen Basis (26) und zumindest einem rechtwinklig hierzu angeordneten Pfeiler (28) ausgebildet ist, wobei auf der Basis (26) zumindest zwei unter einem Winkel zueinander angeordnete Photozeilen (14a, 14b) und eine weitere Photozeile (14c) am Pfeiler (28) vorgesehen sind.

9. Verfahren zur Überprüfung der Positioniergenauigkeit eines Patiententisches (18) in Bezug auf ein Isozentrum (8) eines medizintechnischen Geräts (2) in einem Koordinatenanzeigesystem (10), bei dem ein Prüfkörper (16) in eine Prüfposition gebracht wird, wobei der Prüfkörper (16) nach Art eines auf dem Kopf stehenden Tisches mit einer plattenförmigen Basis (26) und zumindest einem rechtwinklig hierzu angeordneten Pfeiler (28) ausgebildet ist, wobei auf der Basis (26) zumindest zwei unter einem Winkel zueinander angeordnete, aus einer Reihe Photozellen (22) aufgebauten Photozeilen (14a, 14b) und eine weitere aus einer Reihe Photozellen (22) aufgebaute Photozeile (14c) am Pfeiler (28) vorgesehen sind, die Photozeile (14) mit einem von einer Strahlenquelle (12) des Koordinatenanzeigesystems (10) emittierten Prüfstrahl (24) bestrahlt wird und in Abhängigkeit von dem von der Photozeile (14) aufgenommenen Signal (S) die Position des Prüfkörpers (16) relativ zum Isozentrum (8) ermittelt wird.

10. Verfahren nach Anspruch 9,
bei dem die Lage des Signals (S) der Photozeile (14) mit einer Nulllage verglichen wird.

11. Verfahren nach Anspruch 9 oder 10,
bei dem der Prüfkörper (16) aus mehreren Richtungen, insbesondere aus drei Richtungen parallel zu den Achsen des Koordinatenanzeigesystems (10) bestrahlt wird.

12. Verfahren nach einem der Ansprüche 9 bis 11,
bei dem zur Ermittlung der Position relativ zum Isozentrum (8) der Prüfkörper (16) bewegt und das von der Photozeile (14) aufgenommene Signal (S) ausgewertet wird.

## Claims

1. Medical device (2), in particular radiation therapy device, having a patient table (18) which can be positioned in an isocentre (8) and an optical coordinate display system (10), which has at least one radiation source (12), in particular a laser emitter, which is provided to emit a test beam, with a test body (16) for radiation detection with photoelectric lines (14) composed of a row of photoelectric cells (22) being provided to verify the positioning accuracy of the patient table (18), the position of which photoelectric lines is correlated with that of the patient table (18),
**characterised in that**
the test body (16) is embodied in the manner of an upside-down table with a plate-like base (26) and at least one pillar (28) arranged at a right angle thereto, with at least two photoelectric lines (14a, 14b) arranged at an angle to one another on the base (26) and an additional photoelectric line (14c) being provided on the pillar (28).

2. Medical device (2) according to claim 1, **characterised in that** the beam extension (A) of the test beam (24) is dimensioned relative to the photoelectric line (14) such that the test beam (24) only strikes some of the photoelectric cells (22) of the photoelectric line (14).

3. Medical device (2) according to claim 1 or 2, **characterised in that** the photoelectric lines (14a, 14b, 14c) are at a right angle to one another.

4. Medical device (2) according to one of claims 1 to 3, **characterised in that** several photoelectric lines (14a, 14b, 14c) arranged in parallel with one another are provided.

5. Medical device (2) according to claim 3, **characterised in that** the photoelectric lines (14) are arranged on an imaginary circular line.

6. Medical device (2) according to one of the preceding claims, **characterised in that** the test body (16) has a connecting element for precise connection to the patient table (18).

7. Medical device (2) according to one of claims 1 to 5, **characterised in that** the test body (16) can be coupled to a coupling point of an adjusting device (20) of the patient table (18).

8. Test body (16) for verifying the positioning accuracy of a patient table (18) relative to an isocentre (8) of a medical device (2) in a coordinate display system (10), which is embodied in the manner of an upside-down table with a plate-like base (26) and at least one pillar (28) arranged at a right angle thereto, with at least two photoelectric lines (14a, 14b) arranged at an angle to one another on the base (26) and a further photoelectric line (14c) being provided on the pillar (28).

9. Method for verifying the positioning accuracy of a patient table (18) relative to an isocentre (8) of a medical device (2) in a coordinate display system (10), in which a test body (16) is moved into a test position, with the test body (16) being embodied in the manner of an upside-down table with a plate-like base (26) and at least one pillar (26) arranged at a right angle thereto, with at least two photoelectric lines (14a, 14b) composed of a row of photoelectric cells (22) and arranged at an angle to one another on the base (26) and a further photoelectric line (14c) composed of a row of photoelectric cells (22) being provided on the pillar (28), the photoelectric line (14) being irradiated with a test beam (24) emitted by a radiation source (12) of the coordinate display system (10) and the position of the test body (16) relative to the isocentre (8) being determined as a function of the signal (S) picked up by the photoelectric line (14).

10. Method according to claim 9, in which the position of the signal (S) of the photoelectric line (14) is compared with a zero position.

11. Method according to claim 9 or 10, in which the test body (16) is irradiated from several directions, in particular from three directions parallel to the axes of the coordinate display system (10).

12. Method according to one of claims 9 to 11,
in which in order to determine the position relative to the isocentre (8) the test body (16) is moved and the signal (S) picked up by the photoelectric line (14) is evaluated.

## Revendications

1. Appareil de technique médicale (2), notamment appareil de radiothérapie, comprenant une table pour patient (18) positionnable dans un isocentre (8) et comprenant un système optique de visualisation de coordonnées (10), lequel présente au moins une source de radiation (12) ménagée pour l'émission d'un rayon de contrôle, notamment un émetteur laser, un bloc d'essai (16), destiné à détecter la radiation à l'aide de photo-rangées (14) constituées d'une rangée de photocellules (22), étant ménagé pour vérifier la précision de positionnement de la table pour patient (18), la position de ces photo-rangées étant corrélée à celle de la table pour patient (18),
**caractérisé en ce que** le bloc d'essai (16) est réalisé à la manière d'une table placée debout sur la tête, comprenant une base (26) en forme de plateau et au moins un pilier (28) disposé à angle droit par rapport à la base, au moins deux photo-rangées (14a, 14b) disposées dans un angle l'une par rapport à l'autre étant ménagées sur la base (26) et une photo-rangée supplémentaire (14c) étant ménagée sur le pilier (28).

2. Appareil de technique médicale (2) selon la revendication 1,
**caractérisé en ce que** l'étendue de radiation (A) du rayon de contrôle (24) est mesurée de telle manière par rapport à la photo-rangée (14) que le rayon de contrôle (24) tombe seulement sur une partie des photocellules (22) de la photo-rangée (14).

3. Appareil de technique médicale (2) selon la revendication 1 ou 2,
**caractérisé en ce que** les photo-rangées (14a, 14b, 14c) comprennent ensemble un angle droit.

4. Appareil de technique médicale (2) selon l'une quelconque des revendications 1 à 3,
**caractérisé en ce que** plusieurs photo-rangées (14a, 14b, 14c) disposées parallèlement les unes aux autres sont ménagées.

5. Appareil de technique médicale (2) selon la revendication 3,
**caractérisé en ce que** les photo-rangées (14) sont disposées sur un cercle imaginaire.

6. Appareil de technique médicale (2) selon l'une quelconque des revendications précédentes,
**caractérisé en ce que** le bloc d'essai (16) présente un élément de liaison pour la liaison précise avec la table pour patient (18).

7. Appareil de technique médicale (2) selon l'une quelconque des revendications 1 à 5,
**caractérisé en ce que** le bloc d'essai (16) peut être couplé à un point d'accouplement d'un dispositif de réglage (20) de la table pour patient (18).

8. Bloc d'essai (16) destiné au contrôle de la précision de positionnement d'une table pour patient (18) en référence à un isocentre (8) d'un appareil de technique médicale (2) dans un système de visualisation de coordonnées (10), qui est réalisé à la manière d'une table placée debout sur la tête, comprenant une base (26) en forme de plateau et au moins un pilier (28) disposé à angle droit par rapport à la base, au moins deux photo-rangées (14a, 14b), disposées dans un angle l'une par rapport à l'autre, étant ménagées sur la base (26) et une photo-rangée supplémentaire (14c) étant ménagée sur le pilier (28).

9. Procédé destiné à contrôler la précision de positionnement d'une table pour patient (18) en référence à un isocentre (8) d'un appareil de technique médicale (2) dans un système de visualisation de coordonnées (10), dans lequel un bloc d'essai (16) est amené dans une position de contrôle, le bloc d'essai (16) étant réalisé à la manière d'une table placée debout sur la tête, comprenant une base (26) en forme de plateau et au moins un pilier (28) disposé à angle droit par rapport à la base, au moins deux photo-rangées (14a, 14b) constituées d'une rangée de photocellules (22), disposées dans un angle l'une par rapport à l'autre, étant ménagées sur la base (26) et une photo-rangée supplémentaire (14c) constituée d'une rangée de photocellules (22) étant ménagée sur le pilier (28), la photo-rangée (14) étant irradiée par un rayon de contrôle (24) émis par une source de radiation (12) du système de visualisation de coordonnées (10) et la position du bloc d'essai (16) par rapport à l'isocentre (8) étant déterminée en fonction du signal (S) enregistré par la photo-rangée (14).

10. Procédé selon la revendication 9,
dans lequel la position du signal (S) de la photo-rangée (14) est comparée à une position zéro.

11. Procédé selon la revendication 9 ou 10,
dans lequel le bloc d'essai (16) est irradié à partir de plusieurs directions, notamment à partir de trois directions, parallèlement aux axes du système de visualisation de coordonnées (10).

12. Procédé selon l'une quelconque des revendications 9 à 11,
dans lequel, pour déterminer la position par rapport à l'isocentre (8), le bloc d'essai (16) est déplacé et dans lequel le signal (S) enregistré par la photo-rangée (14) est évalué.
